# EUROPEAN PATENT APPLICATION

(11) **EP 1 795 184 A1**
(43) Date of publication of application: **13.06.2007**
(21) Application number: 05026983.6
(22) Date of filing: 09.12.2005
(51) Int. Cl.: A61K 9/16, A61K 9/127, B01J 2/04

(54) **Preparation of powders containing colloidal particles**

(71) Applicant: Ludwig-Maximilians-Universität München, 80359 München (DE); Feyecon Development & Implementation B.V., 1382 GS Weesp (NL)
(72) Inventor: Wiggenhorn, Michael, 80801 München (DE); Winter, Gerhard, 82377 Penzberg (DE); Pellikaan, Hubert, 3572 TT Utrecht (NL)
(74) Representative: Vossius & Partner

(57) **Abstract**

The invention discloses a method for preparing a powder from an aqueous liquid which comprises colloidal particles. The method is based on the use of dense-phase extraction media, such as near-critical or supercritical fluids. The powder obtained through the method can be reconstituted with an appropriate aqueous liquid to yield a colloidal dispersion. The method is particularly useful for drying and stabilising aqueous colloidal dispersions comprising an active compound, such as a drug substance.

## Description

### FIELD OF THE INVENTION

The present invention relates to dry powders and methods of preparing dry powders, and in particular to methods of making dry powders comprising thermally labile components such as colloidal drug carrier systems or thermally labile bioactive compounds. More specifically, the invention relates to methods of preparing such dry powders from aqueous liquids, dry powders obtainable by these methods, to the uses of such powders, and to pharmaceutical compositions comprising the powders.

### BACKGROUND

In the pharmaceutical field, powders are prepared for a variety of applications such as pulmonary and nasal administration, as well as for reconstitution and injection. With respect to aerosols, there are several classes of devices currently available for pulmonary drug administration. These devices include nebulizers (drug in dissolved state aerosolised with compressed air), metered-dose inhalers (MDIs) and dry powder inhalers (DPIs). With this technology colloidal drug carrier systems can be prepared, which can be used as a dry powder or be reconstituted afterwards like ready to use formulations.

Systems like liposomes or solid lipid particles are major examples of colloidal systems. Liposomes are artificial membranes composed of single or multiple phospholipid bilayers enclosing an aqueous compartment. They form spontaneously when phospholipids are placed in an aqueous environment. Liposomes are spherical self-closed structures, composed of curved lipid bilayers, which enclose part of the surrounding solvent into their interior. Hydrophobic drugs and compounds can be incorporated into the lipid bilayers, hydrophilic drugs and compounds can be incorporated within their aqueous cores. Most liposomes are non-toxic, non-antigenic and biodegradable in character since they have the molecular characteristics of mammalian membranes.

However, aqueous liposome dispersions only have limited physical and chemical stability. L Camptothecin acuminata liposomes can aggregate spontaneously and precipitate. Although this sediment may be re-dispersed, the size distribution and the polydispersity can vary from that of the original liposomal dispersion. To a certain extent this can be overcome by incorporation of charged lipids into the liposomes (cationic liposomes). However, the development of a dry powder formulation is more effective.

Liposome formulations are mostly administrated by injection. Present liquid liposome products are not stable. Therefore liposome formulations are subjected to extremely stringent quality criteria, because they can undergo a variety of chemical and physical degradation processes. It is desirable to have final formulations which are stable for six months to two years at room temperature or at refrigeration temperature. Stability requirements have been relaxed by techniques for dehydrating liposomes. Dehydrated liposomes can be distributed to hospitals free of drugs and mixed with the drug immediately prior to use by a hospital pharmacist. However, compounding of the liposome containing drug by a pharmacist increases the cost of the therapy and adds further potential for compounding errors. These factors restrict the use of liposomes as practical carriers of biologically active compounds.

Long term stability of liposome formulations is greatly enhanced when they are stored as dry rather than liquid formulations. A commonly used stabilization method for aqueous liposome suspensions is described in U.S. Pat. Nos. 4,229,360 and 4,247,411. Freeze-drying the liposome components from a suitable organic solvent is described in U.S. Pat. No. 4,311,712. These freeze-dried preparations result in a porous matrix of liposome components which is easily hydrated. Although many formulations have been stabilized with the latter technology, it has some serious drawbacks: it is very time- and energy-consuming and therefore expensive especially in a batch process.

The spray-drying method for preparing a stable liposome precursor in the form of a mixture of spray-dried liposomal components including one or more biologically active compounds which may be stored dry and reconstituted with water to form a liposomal preparation immediately prior to use is described in U.S. Pat. No. 4,830,858. The spray drying process has several difficulties such as selecting limited water content in the solution to be dried. Lipids with heat sensitive functional groups may undergo degradation. Furthermore, drug decomposition may occur.

The spray-freeze drying method can be separated into individual processing steps: spray freezing and lyophilization. The dispersion of preformed frozen droplets is freeze dried by lyophilization. However, the scalability and again the time consuming freeze-drying process are major drawbacks.

Another solution suggested for overcoming the limited physical stability of liposomes is to prepare and store a film of the lipid/biologically active compound and then to disperse the film to form liposomes just prior to administration. However, unit dose film preparation presents serious practical difficulties like the requirements of a container with a high surface area to facilitate solvent evaporation and deposition of a thin film suitable for rapid rehydration to form liposomes readily. This type of container by virtue of its bulk would present severe storage problems.

Solid lipid nanoparticles (SLNs) are an alternative colloidal carrier system to liposomes and other drug carrier systems. An advantage to other colloidal carrier systems is their physical and chemical long term stability. With high pressure homogenisation a very effective production method for SLNs is achievable. SLN dispersions produced by high pressure homogenization (HPH) are characterized by an average size below 500nm and low microparticle content. Other production procedures are based on the use of organic solvents (HPH/solvent evaporation) or on dilution of microemulsions. Optimized SLN are physically stable in an aqueous dispersion for 12 months and recently 24 months. However, in many cases it is highly desirable to freeze dry SLN formulations and for quality acceptable for i.v. administration freeze drying is mandatory.

M. Sarkari et al. described CO2 and fluorinated solvent-based technologies for protein microparticle precipitation from aqueous solutions (Biotechnol. Prog., 19: 448-454 (2003)).

R. T. Bustami et al. provide a review over certain supercritical fluid techniques and applications of such techniques to pharmaceutical powder systems ("Recent application of supercritical fluid technology to pharmaceutical powder systems", Kona, 19: 57-69 (2001).

Further review articles in this field are N. Jovanovic et al. "Stabilization of proteins in dry powder formulations using supercritical fluid technology", Pharm. Res., 11 (21): 1955-1969 (2004) and S. Palakodaty et al. "Phase behavioural effects on particles formation processes using supercritical fluids", Pharm. Res., 16 (7); 976 - 985 (1999).

### BRIEF DESCRIPTION OF THE DRAWINGS

Figure 1 shows the scanning electron micrograph of the particles prepared in Example 1
Figure 2 shows the scanning electron micrograph of the particles prepared in Example 2
Figure 3 shows the scanning electron micrograph of the particles prepared in Example 3
Figure 4 shows the scanning electron micrograph of the particles prepared in Example 4
Figure 5 shows the scanning electron micrograph of the particles prepared in Example 5
Figure 6 shows the scanning electron micrograph of the particles prepared in Example 7
Figure 7 shows the scanning electron micrograph of the particles prepared in Example 8
Figure 8 shows the scanning electron micrograph of the particles prepared in Example 10
Figure 9 shows the scanning electron micrograph of the particles prepared in Example 11
   There is a need for a further method for preparing powders containing colloidal particles which at least partially overcomes one or more of the disadvantages of the known methods. In particular, there is a need for a method which is suitable for the drying of temperature-sensitive colloidal particles regardless of, whether the temperature-sensitivity is associated with the colloidal particles themselves or with an active compound associated therewith.
   It is an object of the present invention to provide such a method. Another object is to provide a method for drying colloidal particles which uses mild conditions, but which requires short processing times and which is cost-effective.
   According to another aspect, it is an object of the invention to provide a powder comprising solid particles, these solid particles comprising colloidal particles such as colloidal drug carrier particles, wherein the colloidal particles and/or any associated active compound may be thermally labile.
   Further objects of the invention will become obvious in the light of the following description and patent claims.

### SUMMARY OF THE INVENTION

According to the invention, a dry powder is prepared from an aqueous liquid which comprises colloidal particles by a method comprising the steps of:
(a) providing an aqueous liquid comprising said colloidal particles and a hydrophilic excipient;
(b) providing a first dense-phase extraction medium which is at least partially miscible with water, and which is a poor solvent for the hydrophilic excipient; and
(c) atomising the aqueous liquid provided in step (a) and contacting it with the first dense-phase extraction medium provided in step (b) while maintaining dense-phase conditions to obtain solid particles.

The method is particularly suitable for converting aqueous dispersions of colloidal drug carrier systems, such as liposomes or lipid complexes, which are themselves sensitive or which may comprise sensitive active ingredients, into dry powders which are stable and which can easily be reconstituted to aqueous compositions. It allows the removal of the solvent under mild conditions which, so that the structure of the colloids and the activity of the active compound can be preserved.

The typical product of the method of the invention is a dry powder comprising solid particles of the hydrophilic excipient, which may be a water-soluble saccharide such as mannitol or trehalose. At least part of these particles individually comprise one or more incorporated colloidal particles, such as one or more liposomes. An active ingredient is incorporated within, or associated with, the colloidal particles. The size of the solid particles is usually selected substantially larger than that of the colloidal particles. For example, the solid particles may have an average particle diameter in the range of about 5 µm to about 100 µm, whereas the incorporated colloidal particles may be in the range of about 20 nm to about 5 µm.

An embodiment of the invention relates to a dry powder obtainable by the above mentioned method.

In a further embodiment of the invention a dry powder is disclosed which comprises solid particles of a hydrophilic excipient, wherein at least part of the particles individually comprise one or more incorporated colloidal particles and a thermally labile active compound.

According to another aspect, the invention provides a dry powder comprising solid particles of a hydrophilic excipient, wherein at least part of said particles individually comprise one or more incorporated thermally labile colloidal particles. The colloidal particles are thermally labile either with regard to the chemical nature of one or more of the constituents of the colloid or to the physical properties of the particles. In a preferred embodiment, the colloidal particles comprise, or are associated with, an e.g. thermally labile, active compound.

The dry powder may be used for the manufacture of a medicament. It may be used as such, or mixed with further ingredients, to form a pharmaceutical composition. Among the preferred types of pharmaceutical compositions are powders for injection or infusion after reconstitution with an appropriate liquid carrier, such as water for injection, or sterile buffer or saline solution.

### DETAILED DESCRIPTION OF THE INVENTION

In a first aspect, the invention provides a method for the preparation of a dry powder from an aqueous liquid which comprises colloidal particles. The method comprises the steps of:
(a) providing an aqueous liquid comprising said colloidal particles and a hydrophilic excipient;
(b) providing a first dense-phase extraction medium which is at least partially miscible with water, and which is a poor solvent for the hydrophilic excipient; and
(c) atomising the aqueous liquid provided in step (a) and contacting it with the first dense-phase extraction medium provided in step (b) while maintaining dense-phase conditions, to obtain solid particles.

A dry powder is a material which comprises a plurality of discrete solid particles. The solid particles may have different sizes, shapes and compositions. Usually, the majority of the solid particles have an average diameter in the in the size range of about 1 µm to about 1 mm. More typically, the solid particles prepared by the method of the invention have an average diameter of about 5 µm to about 500 µm, and more preferably from about 10 µm to about 400 µm. According to another embodiment, the solid particles have an average diameter of about 20 µm to about 300 µm, or from about 30 µm to about 250 µm, or from about 35 µm to about 200 µm.

Average diameters of the particles can be measured and described in various different ways. According to the present invention, an average diameter of the particles refers to the number average diameter of a sample as measured by laser diffraction or an equivalent method, unless stated otherwise. The average diameter of colloidal particles is expressed as a number average diameter of a sample as measured by photon correlation spectroscopy or an equivalent method, unless stated otherwise.

An aqueous liquid is a liquid material which contains water. The material may represent a single liquid phase, or a two- or multiphase system the continuous phase of which is liquid and contains water. Thus, an aqueous suspension or an emulsion the continuous phase of which is aqueous are also examples of aqueous liquids. An aqueous liquid which contains a colloidal material is hereinafter sometimes referred to as an aqueous colloidal dispersion or solution.

Besides water, the aqueous phase of an aqueous liquid may comprise one or more further liquid constituents which are at least partially miscible with water, such as alcohols (e.g. C₁₋₄ alcohols such as ethanol) or ketones (e.g. C₁₋₄ ketones such as acetone). Obviously, any solutes may also be present in the aqueous phase. Preferably the aqueous liquid is water or a mixture of water and ethanol. If the aqueous liquid is a mixture of water and ethanol, the ratio of water:ethanol is preferably 95:5 to 20:80 (v/v), more preferably 85:15 to 40:60 (v/v), most preferably 80:20 to 60:40 (v/v).

Colloidal particles are herein defined as particles of any composition which have a roughly colloidal size range, such as an average diameter of about 10 nm to about 10 µm. More preferably, the colloidal particles in the context of the invention have an average diameter of about 20 nm to about 5 µm, and particularly from about 50 nm to about 1 µm. Other preferred particle sizes depend on the type of colloidal particles, which are used.

The colloidal particles may be solid, semisolid or liquid; however, the inventors are aware that in the lower colloidal size range these terms may not be applicable as the liquid and the solid states may not be distinguishable. Preferably, the colloidal particles used according to the invention are based on lipids, lipoids or amphiphilic compounds. Typically liposomes, lipid complexes, solid lipid nanoparticles, lipoplexes, niosomes, micelles and mixed micelles can be employed.

In one preferred embodiment, the colloidal particles represent association colloids, which means that they are formed as colloidal structures by the self-assembly of the molecules they are composed of. Examples of such colloids are liposomes.

Liposomes are artificial lipid bilayer vesicles of various sizes and structures. Unilamellar vesicles are liposomes defined by a single lipid bilayer enclosing an aqueous space. In contrast, oligo- or multilamellar vesicles comprise several membranes. Typically, the membranes are roughly 4 nm thick and are composed of amphiphilic lipids, such as phospholipids of natural or synthetic origin. Optionally, the membrane properties can be modified by the incorporation of other lipids such as sterols or cholic acid derivatives. Liposomes with particularly flexible membranes based on phospholipids with a low phase transition temperature (i.e. below body temperature) are sometimes referred to as transfersomes.

Depending on their diameter and number of bilayer membranes, liposomes may also be classified as multilamellar vesicles (MLV, two or more bilayers, typically above approx. 150 to 200 nm), small unilamellar vesicles (SUV, one single bilayer, typically below about 100 nm), multivesicular vesicles (MVV, several vesicular structures within a larger vesicle), and large unilamellar vesicles (LUV, one single bilayer, typically larger than about 100 nm).

Particularly useful lipids for the preparation of liposomes include dilauryloyl phosphatidylcholine, dimyristoyl phosphatidylcholine, dipalmitoyl phosphatidylcholine, distearoyl phosphatidylcholine, dioleyl phosphatidylcholine, dipalmitoyl phosphatidylethanolamine, dipalmityloyl sphingomyelin, 1-myristoyl-2-palmitoyl phosphatidylcholine, 1-palmitoyl-2-myristoyl phosphatidylcholine, egg phosphatidylcholine, beef brain sphingomyelin, egg phosphatidylglycerol, dioleoyl phosphatidylglycerol, dipalmitpyl phosphatidic acid, dipalmitoyl phosphatidylserine, phosphatidylinositol, dicetylphosphate, dipalmitoyl phosphatidic acid, stearylamine, 1,2-dioleoyl-3-trimethylammonium-propane, 1,2-dilinoleoyl-sn-glycero-3-phosphoethanol-amine, 1,2-dioleoyl-sn-glycero-3-phosphoethanolamine-n-citraconyl, 1,2-dimyristoyl-sn-glycero-3-phosphocholine, cholesterol, or combinations of any of these, such as phosphatidylcholines with phosphatidylethanolamines or phosphatidylglycerols and sphingolipids.

Liposomes may be loaded with one or more active ingredients and used as drug carrier vehicles in various therapeutic applications. Typically, water-soluble active compounds are encapsulated within the aqueous inner space of the liposomes, whereas lipophilic or poorly water-soluble compounds are associated with the liposome membranes.

Various methods are commonly used to prepare liposomes and to incorporate active ingredients. These include the film method, sonication, detergent dialysis, ethanol injection, ether infusion, reverse phase evaporation, extrusion, and high pressure homogenisation. These methods, the resulting products and their properties are described in more detail e.g. in Kerby et al., Liposomes, in: Encyclopedia of controlled drug delivery, vol. 1, 461-492 (John Wiley & Sons, 1999), which is incorporated herein by reference.

These methods often result in a high level of residuals such as detergents or organic solvents which have to be removed. Methods that avoid the use of organic solvents involve e.g. high pressure extrusion of MLVs through polycarbonate filters of controlled pore size, sonication or high pressure homogenisation of MLVs to SUVs.

Liposomes can be administered orally, transdermally, intravenously, intrabrochially, intramuscularly, intraoculary, subcutaneously and interperitoneally. As a drug delivery system, liposomes can significantly change the pharmacokinetic and pharmacodynamic fate of a compound by enhancing drug uptake, delaying the loss of rapidly cleared drugs and reducing drug toxicity. They have widely been investigated for the delivery of chemotherapeutic agents for treatment of cancer, antimicrobial agents for treatments of bacterial, viral and parasitic diseases, and also for use as immunological adjuvants for delivery of vaccines.

It has surprisingly been found that aqueous liposomal dispersions and other lipid-based colloidal systems may be dried by the method of the invention in such a way that they can easily be reconstituted, usually retaining their approximate particle size and properties. Since the method uses mild conditions, in particular low temperatures, the method is extremely useful for drying thermally labile liposomes.

As used herein, thermally sensitive, temperature-sensitive or thermally labile means that a material is incompatible with drying methods based on the evaporation of water by heat, such as spray drying. In other words, a thermally labile material looses at least some of its structure, activity, functionality or chemical purity when treated by such thermal drying methods, so that the product may be pharmaceutically unacceptable.

Liposomes may be thermally labile in various respects. Their physical structure may be highly sensitive to heat, so that a thermal drying method would lead to the disassembly of the liposomal membranes, to the loss of functionality or to the loss of incorporated or associated active compound. Depending on their composition, they may also be chemically labile to heat. For example, some lipids hydrolyse or oxidise rapidly in an aqueous environment at elevated temperatures.

In a further embodiment, the colloidal particles are non-liposomal colloidal carrier systems for active ingredients, such as lipid complexes, lipoplexes, cochleates, micelles, mixed micelles, or lipid nanoparticles.

Lipid nanoparticles - often called solid lipid nanoparticles (SLN) - are colloidal particles without vesicular structure. They are based on lipids or lipoidal excipients and can be loaded with various lipophilic or poorly water-soluble active compounds. One of the common methods for making these particles is high pressure homogenisation.

Particularly useful lipids and surfactants for composing lipid nanoparticles include triglycerides, such as tricaprin, triiaurin, trimyristin, tripalmitin, tristearin, hydrogenated coco-glycerides, Witepsol W35, -H35, -H42, -E85; glyceryl monostearate, -behenate, -palmitostearate, cetyl palmitate, stearic acid, palmitic acid, decanoic acid, behenic acid, acidan N12; emulsifiers and coemulsifiers such as soybean lecithin (e.g. Lipoid S75, S100), egg lecithin (e.g. Lipoid E80), phosphatidylcholine (e.g. Epikuron 170, - 200), poloxamers (e.g. 188, 182, 407) poloxamine 908, tyloxapol, polysorbates (e.g. 20, 60, 80), sodium cholate, sodium glycocholate, taurocholic acid sodium salt, taurodeoxycholic acid sodium salt, butanol, butyric acid, dioctyl sodium sulfosuccinate, and monooctylphosphoric acid sodium.

For more details on lipid nanoparticles, their production and their uses, reference is made to Muller et al., Adv. Drug Del. Rev. 47:1 (2001), 3-19.

Certain active compounds such as amphotericin B are capable of forming lipid complexes with amphiphilic lipids such as dimyristoyl phosphatidylcholine and dimyristoyl phosphatidylglycerol. Another type of lipid complexes is that of - usually cationic - lipids and nucleic acids such as DNA, RNA or oligonucleotides. Such complexes are often referred to as lipoplexes. The manufacture, properties, and uses of lipid complexes based on various types of lipids are e.g. described in WO 02/072068, which is incorporated herein by reference.

Cochleates are described, for example, in US 4,663,161 and US 4,871,488. These colloidal vehicles usually comprise cationic phospholipid structures that form spiral lipid sheets.

Micelles and mixed micelles are particularly small colloidal structures with various shapes and a length or diameter of typically about 5 to about 100 nm, formed through the association of amphiphilic molecules such as detergents. In contrast to liposomes, these structures are based on monolayers which are less stable and tend to disassemble upon dilution. Micelles are e.g. disclosed in WO 02/085337 and EP-A 730 860, the teachings of which are incorporated herein by reference.

Niosomes are colloidal structures similar to liposomes, except that the vesicle membranes are predominantly composed of nonionic amphiphilic compounds instead of ionic components.

According to the method of the invention, step (a) requires that an aqueous liquid is provided which comprises the colloidal particles and a hydrophilic excipient. The hydrophilic excipient functions as a carrier material of the powder particles in which the colloidal particles are incorporated. The colloidal particles are preferably contained in the aqueous liquid in an amount of 0.1 to 40 mM, more preferably 0.2 to 20 mM, most preferably 0.5 to 10 mM.

As used herein, a hydrophilic excipient is a pharmaceutically acceptable, pharmacologically substantially inert material with hydrophilic properties. Hydrophilicity means that the hydrophilic excipient should be substantially water soluble. Preferably, its water solubility is at least about 1 wt.-% at 25 C, and more preferably at least about 2 wt.-%. In other embodiments, the water solubility is at least about 5 wt.-% or even at least about 10 wt.-%. At least a substantial portion, if not all, of the excipient should preferably be provided in dissolved form. The water solubility can be important for processing reasons and for ensuring good reconstitution properties of the powder prepared by the method of the invention.

Useful hydrophilic excipients can be monomeric, oligomeric or polymeric and may be found among several chemical classes of compounds. According to one of the preferred embodiments, the hydrophilic excipient is a saccharide. A saccharide, or carbohydrate, is defined a compound predominantly composed of carbon, hydrogen, and oxygen. Useful saccharides include sugar and sugar alcohols, oligosaccharides, water soluble polysaccharides and derivatives thereof. Preferred saccharides according to the invention include glucose, fructose, lactose, sucrose, trehalose, maltose, cellobiose, galactose, maltotriose, maltopentose, raffinose, dextrin, dextran, inulin, mannitol, sorbitol, xylitol, chitosan; water soluble cellulose derivatives such as methylcellulose, hydroxypropylcellulose, hydroxyethylcellulose, and hypromellose; alginates, soluble starches or starch fractions, xanthan gum, guar gum, pectin, carrageen, galactomannan, gellan gum, tragacanth, including any derivatives of these. Particularly preferred saccharides, at present, are glucose and trehalose.

Other useful hydrophilic excipients may be selected from other chemical classes, such as from water soluble amino acids, peptides or proteins. For example, glycine or other natural amino acids may be used. Useful proteins include gelatine, albumin, whey protein, soy protein, or other food or vegetable proteins.

Still further examples of useful hydrophilic excipients are polymers such as water soluble polymers such as solid polyethylene glycols, polyvinylalcohol, polyacrylates, or polyvinylpyrrolidone.

Optionally, mixtures of more than one hydrophilic excipient can be used. For example, there may be a need to adjust several parameters such as pH, solubility, and wettability independently. In this case, a first hydrophilic excipient may be selected as a basic carrier material for the colloidal particles, whereas one or more further hydrophilic excipients may be incorporated to obtain a certain pH and/or wettability.

The content of the hydrophilic excipient, or mixture of hydrophilic excipients, in the aqueous phase may vary widely, depending on its aqueous solubility and other considerations, and be even up to about 80 wt.-%. More preferably, it is from about 0.1 to about 65 wt.-%. A content of about 3 to about 60 wt.-%, and particularly from about 5 to about 50 wt.-% is presently preferred.

Of course, the aqueous liquid may comprise further excipients or auxiliary substances, which may or may not be hydrophilic or water soluble. Depending on their nature and that of the extraction medium, i.e. on whether or not these substances are soluble in and extracted by the extraction medium, such substances can be incorporated into the powder or removed together with the water. Substances, which are incorporated into the powder, should be pharmaceutically acceptable.

Excipients which may be useful in powder formulations, and in particular in powder formulations which are used for reconstitution and optionally, parenteral administration, are generally known to pharmaceutical formulation scientists. Potentially useful excipients include stabilisers, surfactants, wetting agents, bulking agents, lyophilisation aids, antioxidants, chelating agents, preservatives, osmotic agents, acidic or alkaline excipients for adjusting the pH, etc.

Among the preferred excipients according to the invention are stabilisers and antioxidants. Antioxidants may prevent the oxidation of an incorporated active compound, but also that of components of the colloidal carrier particles, in particular if lipids are used which are sensitive to oxidisation. Useful compounds include, for example, lipid-soluble antioxidants such as alpha-, beta-, and gamma-tocopherol, ubiquinol, lycopene, alpha- and beta-carotene, nordihydroguaiaretic acid, butyl hydroxyanisole, butyl hydroxytoluene, ethylenediamine tetraacetic acid, diethylenetriamine pentaacetic acid, desferal, p-hydroxybenzoic, vanillic, syringic, 3,4-dihydroxybenzoic, p-coumaric, ferulic, sinapic and caffeic acids, ascorbyl palmitate, carnosol, esculetin, esculin, fraxetin, fraxin, quercetin, morin, etc. Particularly preferred are alpha-tocopherol and ethylenediamine tetraacetic acid, including the pharmaceutically acceptable derivatives thereof. On the other hand, if chemically pure, semisynthetic or synthetic saturated lipids are used for composing the colloidal particles, no antioxidant may be needed.

In step (b) of the method of the invention a first dense-phase extraction medium which is at least partially miscible with water, and which is a poor solvent for the hydrophilic excipient is provided. The step can be conducted at a time, which is selected independently of the step of providing the aqueous liquid.

As used herein, the term "dense-phase extraction medium" means a medium which is a compressed gas i.e. the medium is gaseous at ambient conditions but is employed in the method of the present invention under pressure. Accordingly, dense-phase extraction media include compressed gases, near-critical gases and supercritical gases.

By controlling temperature and pressure, most substances that are gaseous at ambient conditions can be set into a state which is different from the common solid, liquid and gas states. In this state supercritical fluids are effective and selective solvents. A supercritical fluid is defined as a substance above its critical temperature T_{c} and critical pressure p_{c}, which together define the critical point in the phase diagram. The critical point represents the highest temperature and pressure at which the substance can exist as a vapour and liquid in equilibrium. The near-critical region can be defined as a region below the critical pressure and/or temperature. Within the near-critical region a fluid can exist in a state of two phases, with different densities for the vapour and the liquid phase. An explanation of supercritical and near-critical regions as well as the critical parameters of certain compounds can be found in R. T. Bustami et al., "Recent application of supercritical fluid technology to pharmaceutical powder systems", Kona, 19, page 58 (2001). For example, in the near-critical region the temperature can be from 1.0 x T_{c} to 1.2 x T_{c} while the pressure can be from 0.9 x p_{c} to 2.0 x p_{c}.

But even below their critical pressure, i.e. at or even below near-critical conditions, certain compressed gases may attain solvent and penetration properties, which are highly useful in extraction, precipitation, and drying processes. Thus, the extraction medium is provided in the dense-phase state, and optionally in the near- or supercritical state. The near-critical region starts e.g. for CO₂ around 15°C below the critical point and at around 35 bar and has strong effects on physicochemical properties and phase behaviour.

Particularly useful extraction media according to the invention are compressed gases, i.e. substances or mixtures of substances, which are gaseous at ambient conditions (e.g. 25°C and 1 atm). Examples of potentially useful gases include air, nitrogen, carbon dioxide, ethane, propane, nitrous oxide, argon, oxygen, methane, butane, n-pentane, nitrous oxide, sulphur hexafluoride, chlorofluorocarbons, fluorocarbons, ethers comprising two alkyl radicals which may be the same or different and which contain no more than 3 carbon atoms, carbon monoxide, helium, hydrogen, xenon, including mixtures of any of these. Particularly preferred gases are carbon dioxide, ethane, argon, xenon, air, and nitrogen, and mixtures of any of these. The presently most preferred gas is carbon dioxide.

The extraction medium is, with regard to its composition and state, selected to be at least partially miscible with water, which is understood herein as being capable of dissolving at least about 0.01 wt.-% water, and more preferably at least about 1 wt.-% or even at least about 5 wt.-% water. Higher degrees of miscibility, such as substantial or even full miscibility, represent another preferred embodiment. After all, the primary function of the extraction medium is the selective removal of water from the aqueous liquid provided in step (a) of the method of the invention.

At the same time, the extraction medium is selected to be a poor solvent for the hydrophilic excipient. This means that the solubility of the hydrophilic excipient in the extraction medium should be low, and preferably less than about 1 wt.-%, and more preferably less than about 0.5 wt.-%. According to another embodiment, the solubility in the extraction medium should be less than about 0.1 wt.-%.

The extraction properties of the extraction medium may be further adjusted by adding an organic cosolvent to it. Preferably, the cosolvent is a C₁₋₄ alcohol, a C₁₋₄ ketone or a C₁₋₄ aldehyde. Examples of potentially useful cosolvents include methanol, ethanol, n-propanol, isopropanol, dichloromethane, acetone, acetonitrile, acetic acid, dimethyl ether, and diethyl ether. The presently most preferred cosolvent is ethanol.

The content of the cosolvent in the extraction medium may be up to about 50 wt.-%. More preferably, the content is in the range of about 0.1 to about 30 wt.-%. According to another preferred embodiment, the extraction medium comprises carbon dioxide and about 2 to about 10 wt.-% ethanol.

If a cosolvent is used, it may be introduced together with the first dense-phase extraction medium, or together with the aqueous liquid comprising the colloidal particles and the hydrophilic excipient. As a further alternative, it may be introduced separately, such as by atomisation, and thereby contacted with the extraction medium and/or the aqueous liquid.

The amount of extraction medium should be selected taking into account the type and relative amount of the extraction medium, the cosolvent if used, and the amount of aqueous liquid to be dried. Typically, the weight ratio of the aqueous liquid to the first dense-phase extraction medium is from 0.1 : 0.99 to 90 : 10. In one of the preferred embodiments, the weight ratio is selected in the range of about 1:2 to 1:10.

The method further includes the step (c) of atomising the provided aqueous liquid and contacting it with the provided first dense-phase extraction medium while maintaining dense-phase conditions to obtain solid particles. Obviously, this step is conducted after the steps of providing the aqueous liquid and of providing the first dense-phase extraction medium.

As used herein, atomising refers to the conversion of a material into fine particles or droplets. This can be done in various different ways. For example, liquid or liquid-like materials such as those provided in steps (a) and (b) can be atomised by spraying them through a nozzle. Unless the atomisation takes place within a continuous liquid or liquid-like material, the term spraying may be used interchangeably with atomising.

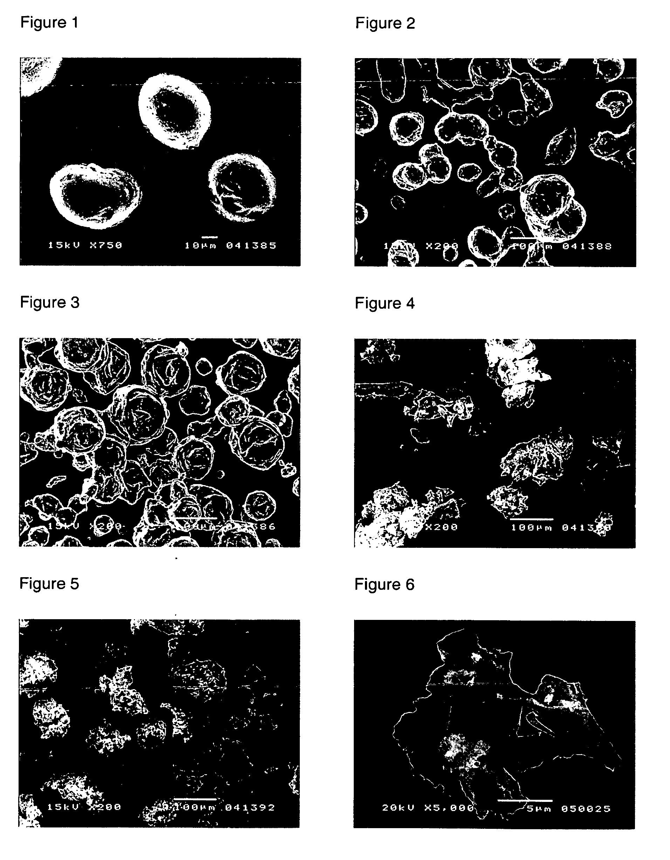

According to the invention, the aqueous liquid comprising the colloidal particles and the hydrophilic excipient is atomised into fine droplets, whereas the first dense-phase extraction medium may or may not be atomised. If the first dense-phase extraction medium is atomised, it is preferred that it is atomised concurrently with the aqueous liquid, such as via a two-fluid nozzle. This will also ensure that immediately upon atomisation, the aqueous liquid and the first dense-phase extraction medium will be in contact with each other, so that the first dense-phase extraction medium can extract the water from the aqueous liquid, leading to the formation of solid particles. Alternatively, a vessel or reactor may be pre-filled with the first dense-phase extraction medium, and the aqueous liquid may be subsequently sprayed into the vessel. Both variants may be understood as a modified spray drying process using dense-phase media, the principles of which are generally known and, for example, described in Jovanovic et al., Pharmaceutical Research, 21:11 (2004), 1955-1969, the disclosure of which is incorporated herein by reference. Other possible experimental set-ups are discussed in R. T. Bustami et al., "Recent application of supercritical fluid technology to pharmaceutical powder systems", Kona, 19, pages 60 - 61 (2001) and N. Jovanovic et al., "Stabilization of proteins in dry powder formulations using supercritical fluid technology", Pharm. Res., 11 (21), pages 1957 - 1958 and 1961 - 1962 as well as Figures 4 and 5 (2004).

Useful atomising nozzles to carry out this step of the method of the invention are also generally known to the skilled worker in the field. They include, for example, rotating disk nozzles, impinging jet nozzles, capillary nozzles, single orifice nozzles, ultrasonic nozzles of vibrating or pulsating type, two-fluid nozzles such as coaxial two-fluid nozzles etc.

An essential feature of this process step is that the dense-phase conditions are maintained during atomisation. Dense-phase conditions, including supercritical conditions, must be defined relative to the respective extraction medium. As a general rule, however, the pressure should be selected in the range of about 10 to about 300 bar. Typically the temperature should be selected in the range of about 0 to about 60 °C.

More preferably, the pressure is selected in the region of about 10 to about 140 bar. In the case of an extraction medium predominantly comprising carbon dioxide as a compressed gas, it is preferred to select a relatively low pressure within this range, such as about 10 to about 40 bar, and more preferably about 15 to about 30 bar. In the case that at least about 2 wt.-% of ethanol are added as cosolvent, a higher pressure should preferably be selected, such as about 70 to about 140 bar, or from about 80 to about 120 bar. However, the use of other compressed gases than carbon dioxide may necessitate the selection of different pressures.

According to another preferred embodiment of the invention, the pressure is maintained at a substantially constant level while the step of atomising the aqueous liquid and contacting it with the extraction medium is conducted. As used herein, a substantially constant pressure means that the pressure does not change by more than about 3 bar from the mean pressure. More preferably, the pressure is kept even more constant, such as not exceeding fluctuations of about 1 bar. Apparatuses including vessels and pumps, which are suitable for conducting the method step and maintaining the described conditions, are generally known to the expert in the field.

As mentioned, the temperature is generally selected in the range of about 0 to about 60 °C. More preferably, the temperature is selected in the range of about 25 to about 50 °C, such as about 30 °C, 35 °C, 40 °C, or 45 °C. This preference applies in particular to embodiments in which carbon dioxide is selected as the first dense-phase extraction medium, or as the compressed gas component of the first dense-phase extraction medium.

Atomising the aqueous liquid and contacting it with the first dense-phase extraction medium under the conditions specified above leads to the removal of water, i.e. to the drying of components contained in the aqueous liquid, and to the formation of solid particles of the hydrophilic excipient wherein the colloidal particles are dispersed or embedded. The solid particles, which typically represent a dry powder, may be collected after reducing the pressure to approximately normal conditions.

Typically, the solid particles, or the powder comprising or consisting of the solid particles, are substantially dry. If the process is conducted for a sufficiently long time or using a sufficient amount of first dense-phase extraction medium for the selected amount of aqueous liquid to be dried, a residual water content of not more than about 7 wt.-%, and typically not more than about 5 wt.-% is easily achievable, and such a water content is preferred. More preferably, the residual water content of the powder is not more than about 4 wt.-%, and according to further embodiments not more than about 3 wt.-% and not more than about 2 wt.-%, respectively.

In certain cases, in particular if an organic cosolvent is used as a component of the first dense-phase extraction medium, it may be useful to perform an additional step subsequent to steps (a) to (c) in order to remove residual cosolvent from the particles that have been obtained. In particular, it may be useful to contact the solid particles with a second dense-phase extraction medium, which does not contain any organic cosolvent. Obviously, the second dense-phase extraction medium should - like the first dense-phase extraction medium - be a poor solvent for the hydrophilic excipient. It may or may not be miscible with water, but it should be at least partially miscible with the cosolvent of the first dense-phase extraction medium. For example, if the first dense-phase extraction medium is selected to be carbon dioxide comprising ethanol as a cosolvent, the second dense-phase extraction medium may simply be carbon dioxide.

In order to obtain solid particles in which colloidal particles are incorporated or embedded, it is useful to select a weight ratio of the hydrophilic excipient to the colloidal particles which favours an acceptable degree of incorporation of the colloid. For example, if the aqueous liquid comprises more colloidal particles than hydrophilic excipient, some of te colloidal particles may not be incorporated in or embedded in the solid particles produced by the method of the invention. Therefore, it is preferred that the weight ratio of the hydrophilic excipient to the colloidal particles is at least about 1, and more preferably at least about 2, such as about 3 to about 5 or even more. On the other hand, an extremely high ratio should be avoided because it might lead to rather large powder volumes for a specific content of colloidal particles and thus to a large volume of liquid composition after reconstitution. Thus, ratios of more than about 50 or even more than 100 are presently less preferred, and ratios of less than about 50 and particularly of less than about 20 are considered more useful.

As pointed out, the method of the invention allows the conversion of an aqueous liquid containing colloidal particles into a dry powder. The solid particles comprise a hydrophilic excipient, which acts as a carrier for the colloidal particles which are embedded or incorporated in the solid particles. The method is conducted at moderately low temperatures, so that it is particularly advantageous for the drying and stabilisation of colloidal particles which are thermally labile or which comprise a thermally labile active agent. In contrast to known methods of drying such thermally labile colloidal particles, the most common of which is lyophilisation, the method of the invention requires little time and promises to be very cost-effective. Furthermore, it can be conducted as a small batch process, but also as a semi-continuous or continuous process. In contrast to lyophilisation which often leads to a porous, coherent solid unit, the method of the invention leads to a free-flowing powder which may be easily filled into primary packaging containers by standard - optionally aseptic - powder fill processes. Through its unique combination of features, it is assumed that it represents the only drying method by which a dry powder of solid particles of a hydrophilic excipient comprising incorporated thermally labile colloidal particles can be prepared. The same advantage of the method of the invention over spray freeze drying is that lyophilisation, which is one step during spray freeze drying, is avoided.

Again, the colloidal particles which are incorporated into excipient particles by using the method of the invention preferably represent drug carrier systems or comprise an active compound. As used herein, an active compound is a compound, or mixture of compounds, which has a particular bioactivity based on which it is useful as an agent useful for the diagnosis, prevention, or treatment of a human or animal disease or condition. Drug substances, diagnostic compounds and vaccines are important examples of active compounds according to the present invention.

The active compound may be incorporated within or associated with the colloidal particles. In the case of liposomes, for example, a hydrophilic active compound - perhaps with the exception of some large proteins - may be encapsulated within the aqueous inner space of the liposomes, whereas a lipophilic active compound is usually associated with the membrane-forming lipids. In the case of lipid nanoparticles, on the other hand, these rules of thumb do not apply as lipophilic compounds are easily incorporated within the matrix of such nanoparticles.

Colloidal particles are particularly suitable drug carriers for active compounds which are not easy to deliver effectively, such as very poorly soluble compounds, sensitive (including thermally labile) active compounds, peptides, proteins, and nucleic acids including DNA, RNA, iRNA, siRNA, or oligonucleotides. Another preferred group of active compounds are those which are more effectively delivered in colloidal form because the distribution of colloids in the organism after intravenous injection is more favourable with regard to efficacy or side effects than the administration of a solution of the compound. This is the case with certain antimicrobial, antifungal, antiviral, and cytostatic or cytotoxic agents. Examples of such agents include doxorubicin, mitoxanthrone, and amphotericin B.

In one of the embodiments, the invention involves cationic liposomes. Cationic liposomes can be successfully used as a drug delivery vehicle for some hydrophobic drugs. Among the preferred hydrophobic drugs are taxoids, camptothecins, doxorubicin, michellamine B, vincristine, and cisplatin. The term "taxoid" is used to refer to paclitaxel, cephalomannine, baccatin III, 10-deacetyl baccatin III, deacetylpaclitaxel and deacetyl-7-epipaclitaxel and derivatives and precursors thereof. Paclitaxel is one example of a taxoid. Paclitaxel, also known as TAXOL^{™} is a diterpene plant product derived from the western yew Taxus brevifolia. This drug, currently in clinical trials, has exhibited a striking 30 to 40% response rate against advanced cases of ovarian and a number of other cancers. Currently, paclitaxel is extracted with organic solvents from the milled bark of T. brevifolia.

Camptothecin is derived from a tree, Camptotheca acuminata. As used herein, the term "camptothecins" refers to camptothecin and its methoxylated analogs and carboxylated analogs and precursors which have similar biological activity and hydrophobic properties. Camptothecin is also utilized for the treatment of cancers.

Like paclitaxel, camptothecins are water insoluble, heat sensitive and in short supply. The sodium salt of camptothecin has been utilized in initial clinical studies. Recent clinical trials suggest that several camptothecin analogs may have activity against a broad range of human/animal tumours. This composition is believed to act as an inhibitor the enzyme topoisomerase I.

The solid particles, or the powder comprising the same, as obtained by the method of the invention may be used in the manufacture of a medicament or a diagnostic product. If the powder fulfils all requirements of a pharmaceutical dosage form, it may be used as such, and filled directly into appropriate primary packaging containers. Alternatively, the powder may be further processed. For example, it may be mixed with further active and/or inactive ingredients, or it may be compressed into a pharmaceutical tablet.

Preferably, the powder is used - either alone or with further constituents - for the manufacture of a pharmaceutical composition which can be in powder form, in particular in the form of a sterile powder for reconstitution with an appropriate aqueous medium.

After the reconstitution of powders containing colloidal systems, such as with water for injection or other buffer systems, the resulting formulations can be used for parenteral (e.g. i.v. or locoregional) injection, oral administration, pulmonary or nasal inhalation. Solvents, in case they are used in the preparation method, will be extracted during the method of invention below permissible or detectable limits, which is a particular advantage of the invention over methods known in the prior art.

### EXAMPLES

The following examples are meant to further illustrate the invention and some of its preferred embodiments without limiting its scope. Further examples and embodiments will easily be derived from the description, optionally in combination with generally known technical information, in particular in combination with known principles of designing and using colloidal drug carrier systems and supercritical fluid technology.

### Liposome preparation

Cationic liposomes with a total lipid content between 10 to 40mM were prepared by ethanol injection and extrusion through a polycarbonate membrane. DOTAP (1,2-dioleoyl-3-trimethylammonium-propane) and DOPC (1,2-dioleoyl-sn-glycero-3-phosphocholine), both from Avanti Polar Lipids (Alabaster, USA), were dissolved in ethanol. Multilamellar liposomes formed spontaneously upon the injection into 10% trehalose solution (Ferro Pfanstiehl, USA).

### Analytics of the liposomes

The particle size (Z-average) and polydispersity (PI) of the liposomes were analyzed by photon correlation spectroscopy (PCS) using a Malvern Zetasizer Nano (Malvern Instruments; UK). The lipid concentration of DOTAP and DOPC was analyzed by HPLC using an UV/VIS detector at 205 nm. Separation and quantification of the components was carried out using a C8 LiChrospher 60 RP-selected B column (250 x 4mm, 5 µm particle size) with a C18 pre-column.

### Particle preparation

In the experimental set-up an aqueous solution containing liposomes is dispersed into near- or supercritical carbon dioxide. The suspension was atomized over a nozzle surrounded by the supercritical gas and the co-solvent which are fed continuously into the precipitation pipe and in the vessel using syringe pumps. After mixing the aqueous suspension with the CO₂/ethanol the phase splitting occurs and precipitation induced by phase separation takes place. The particle size can be controlled over the increase of atomization forces. The drying process is completed by a final rinse of the particles with CO₂ after the spraying-process.

### Analytics of particles

Images of the product were obtained using scanning electron microscopy (SEM) (Joel, JSM 5400, Japan) to study the morphology. Residual moisture was determined by a coulometric Karl Fischer titrator with a head-space oven (Analytic Jena AG, Germany). Particle size distribution was measured with a He-Ne laser beam equipped laser diffraction analyzer (Mastersizer X, Malvern, Germany). Residual solvent concentration was obtained by GC-headspace chromatography (Perkin Elmer Autosystem and Perkin Elmer HS40 XL, USA).

### Example 1

A vessel with a volume of 1 litre was filled with carbon dioxide to a pressure of 100 bar at 37.5 °C. At the bottom of this vessel, a filter was mounted to enable the harvesting of the solid particles to be produced. In addition, an aqueous dispersion of preformed liposomes was prepared with a lipid concentration of 10 mM and a trehalose content of 10 wt. %. The number average diameter of the liposomes was about 220 nm, with a polydispersity of about 0.2. During 10 min, the aqueous dispersion was sprayed into the vessel through a nozzle having an orifice of 0.1 mm diameter at a 1 ml/min rate. Simultaneously an additional amount of 200 g/min carbon dioxide and 20 ml/min ethanol were fed coaxially around the nozzle spraying the suspension. The pressure was maintained at 100 bar ± 1 bar by means of a backpressure valve.

After 10 minutes, the introduction of the liposome dispersion and of the carbon dioxide/ethanol extraction medium was stopped. Subsequently, the vessel was flushed with 5000 g of carbon dioxide without ethanol to remove all residual solvent. Then the pressure was slowly reduced to atmospheric conditions. The vessel was opened and a powder of solid particles was obtained. The residual moisture content was below 5 %. The size of the smooth and hollow powder particles was in the range of 10 to 150 µm. After reconstitution with water for injection a liposomal dispersion was obtained. The number average particle diameter was about 165 nm, with a polydispersity of 0.38. The residual ethanol concentration was 1280 ppm.

### Example 2

The method was conducted in analogy to example 1, except that an aqueous liposome dispersion of 20 mM and a trehalose concentration of 15 wt. % were used at 36.1°C. Ethanol was added to the CO₂ flow (20ml EtOH / 190g CO₂). A dry powder was obtained with particle sizes varying between 20 to 160 µm. The residual moisture content was 1.1 wt.-%. After reconstitution of the powder, liposomes were obtained with a number average diameter of 190 nm and a polydispersity of 0.48, compared to a number average diameter of 200 nm and a polydispersity of 0.5 before drying.

### Example 3

The method was conducted in analogy to example 1, except that an aqueous liposome dispersion of 40 mM and a trehalose concentration of 10 wt. % were used at 36.9°C. Ethanol was added to the CO₂ flow (20ml EtOH / 210g CO₂). The number average diameter of the liposomes was about 250 nm, with a polydispersity of about 0.45. A dry powder was obtained with hollow particles, the sizes varying between 20 to 200 µm with a residual moisture of 4.8 %. After reconstitution of the powder, liposomes were obtained with a number average diameter of 220 nm and a polydispersity of 0.43.

### Example 4

The method was conducted in analogy to example 1, except that an aqueous liposome dispersion of 20mM and a trehalose concentration of 40 wt. % were used at 36.8°C. Ethanol was added to the CO₂ flow (20ml EtOH / 205g CO₂). A dry powder was obtained with particle sizes varying between 40-180 µm.

### Example 5

The method was conducted in analogy to example 1, except that an aqueous liposome dispersion of 10mM and a trehalose concentration of 40 wt. % were used 36.9°C. Ethanol was added to the CO₂ flow (20ml EtOH / 180g CO₂). A dry powder was obtained with particle sizes varying between 10 to 180 µm and a residual moisture content of 2.1 %.

### Example 6

The method was conducted in analogy to example 1, except that an aqueous liposome dispersion of 10mM and a trehalose concentration of 60 wt. % were used at 36.7°C. Ethanol was added to the CO flow (20ml EtOH / 190g CO₂). A dry powder was obtained with particle sizes varying between 50 to 200 µm.

### Example 7

The method was conducted in analogy to example 1, except that an aqueous liposome dispersion of 10mM and a trehalose concentration of 10 wt. % were used at 39.9°C. Ethanol was added to the CO₂ flow (20ml EtOH / 210g CO₂). A dry powder was obtained with particle sizes varying between 2 to 100 µm and a residual moisture content of 3.7 %. After reconstitution of the powder, liposomes were obtained with a number average diameter of 161 nm and a polydispersity of 0.31, compared to a number average diameter of 220 nm and a polydispersity of 0.20 before drying.

### Example 8

The method was conducted in analogy to example 1, except that an aqueous liposome dispersion of 10mM and a trehalose concentration of 10 wt. % were used at 40°C. Ethanol was added to the CO₂ flow (20ml EtOH / 205g CO₂). The aqueous dispersion was fed into the vessel with a 0.63 ml/min rate. The vessel was flushed with 6000 g of carbon dioxide to remove all residual solvents. A dry powder was obtained with particle sizes varying between 5 to 100 µm and a residual moisture content of 2.1 %.

### Example 9

The method was conducted in analogy to example 1, except that an aqueous liposome dispersion of 10mM and a trehalose concentration of 10 wt. % were used at 40.5°C. Ethanol was added to the CO₂ flow (20ml EtOH / 205g CO₂). A dry powder was obtained with particle sizes varying between 10 to 200 µm.

### Example 10

The method was conducted in analogy to example 1, except that an aqueous liposome dispersion of 10mM and a trehalose concentration of 10 wt. % were used at 38.1°C. Ethanol was added to the CO₂ flow (20ml EtOH / 210g CO₂). The aqueous dispersion was fed into the vessel with a 0.5 ml/min rate. A dry powder was obtained with particle sizes varying between 5 to 100 µm, with a residual moisture 6.1 %. After reconstitution of the powder, liposomes were obtained with a number average diameter of 194 nm and a polydispersity of 0.25, compared to a number average diameter of 220 nm and a polydispersity of 0.30 before drying.

### Example 11

The method was conducted in analogy to example 1, except that an aqueous dispersion of liposomes was prepared with a lipid concentration of 10mM and a trehalose content of 10 wt. %. The number average diameter of the liposomes was about 220 nm, with a polydispersity of about 0.2. A higher ethanol supersaturation in the reaction vessel was achieved (30ml EtOH / 200g CO₂). The vessel was flushed with 6000 g of carbon dioxide to remove all residual solvents. Dry powder was obtained with hollow particles, the size varying between 20 to 150 µm, with a residual moisture of 4.7 %. After reconstitution of the powder, liposomes were obtained with a number average diameter of about 140 nm and a polydispersity of 0.31. The residual ethanol concentration was not detectable by GC-head space chromatography.

### Example 12

The method was conducted in analogy to example 1, except that an aqueous dispersion of liposomes was prepared with a lipid concentration of 20mM and a glucose content of 15 wt. %. A lower ethanol supersaturation in the reaction vessel was achieved (10ml EtOH / 200g CO₂). Dry powder was obtained, the size varying between 20 to 200 µm, with a residual moisture of 4.8 %. After reconstitution of the powder, liposomes were obtained with a number average diameter of about 260nm and a polydispersity of 0.4.

### Example 13

The method was conducted in analogy to example 1, except that an aqueous solid lipid nanoparticles dispersion of 5 mM and trehalose concentration of 10 wt % were used. A dry powder was obtained with particle sizes of 50 to 120 µm with a residual moisture of 2.5 wt.-%. After reconstitution of the powder, a dispersion of solid lipid nanoparticles was obtained with a number average diameter of 210 nm and a polydispersity of 0.25, compared with a number average diameter of 220 nm and a polydispersity of 0.2 before drying.

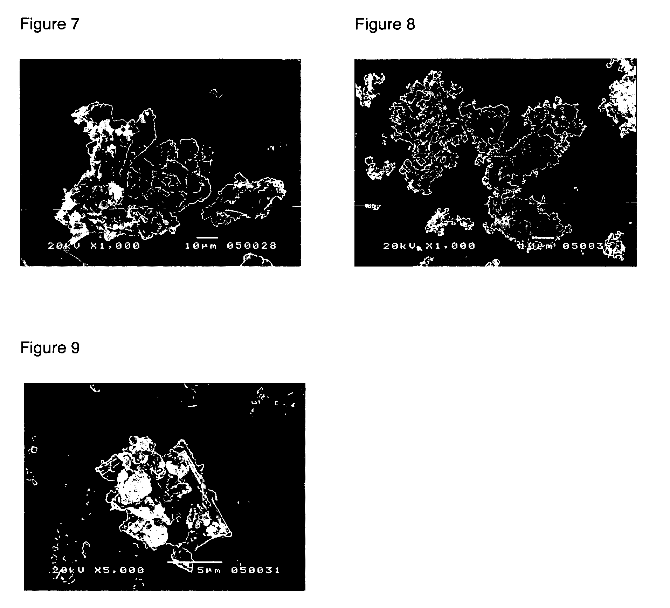

Particles obtained in Examples 1 to 5, 7, 8, 10 and 11 were imaged using a scanning electron microscopy. The images are shown in Figures 1 to 9.

The results obtained in Examples 1 to 13 can be evaluated as follows.
- The addition of a cosolvent such as ethanol increases the miscibility of water in CO₂ and leads to improved extraction forces.
- With the process conditions selected in Examples 1 to 3 spherical single particles are produced due to the favourable ethanol/CO₂ ratio in combination with the ethanol / solution flow of 20 ml/min / 1 ml/min which can enhance precipitation. A higher ethanol / solution flow (Examples 8 and 10) and a lower ethanol / CO₂ flow (Example 7) inhibit droplet break-up and cause agglomeration.
- The average diameter of the particles can be controlled by atomization of CO₂ within the pipe and vessel.
- The residual moisture neither correlates with the particle size and shape nor with the different flow-rates.
- In all of the examples, the size of the liposomes after reconstitution was smaller than that of the liposomes before they were subjected to the method of the present invention. The polydispersity values indicate that the liposomes can be recovered after drying.
- Quantitative lipid analytics by HPLC showed only a minor decrease in DOTAP and DOPC concentration. Phospholipids are poorly soluble in supercritical carbon dioxide. Using ethanol as a cosolvent can slightly increase the solubilities of the lipids. However, the residual solvent concentration determined by GC-headspace chromatography showed lower values for liposomes dried with supercritical fluids compared to those for freeze-dried liposomes. This can be explained by the high miscibility of ethanol and CO₂.

## Claims

1. A method for the preparation of a dry powder from an aqueous liquid which comprises colloidal particles, said method comprising the steps of:
(a) providing an aqueous liquid comprising said colloidal particles and a hydrophilic excipient;
(b) providing a first dense-phase extraction medium which is at least partially miscible with water, and which is a poor solvent for said hydrophilic excipient; and
(c) atomising the aqueous liquid provided in step (a) and contacting it with the first dense-phase extraction medium provided in step (b) while maintaining dense-phase conditions to obtain solid particles.

2. The method of claim 1, wherein the first dense-phase extraction medium and/or the aqueous liquid further comprise(s) an organic cosolvent.

3. The method of claim 2, wherein the content of the organic cosolvent in the first dense-phase extraction medium and/or in the aqueous liquid is from about 0.1 to about 30 wt.-%, and particularly from about 2 to about 10 wt.-%.

4. The method of claim 2 or 3, wherein the first dense-phase extraction medium further comprises an organic cosolvent and wherein the method further comprises the step of (d) combining the solid particles obtained in step (c) with a second dense-phase extraction medium which is at least partially miscible with the cosolvent of the first dense-phase extraction medium provided in step (b), and wherein the second dense-phase extraction medium is a poor solvent for the hydrophilic excipient.

5. The method of any of the preceding claims, wherein step (c) is carried out by simultaneously spraying the aqueous liquid provided in step (a) and the first dense-phase extraction medium provided in step (b), and/or by spraying the aqueous liquid provided in step (a) into a vessel containing the first dense-phase extraction medium.

6. The method of any of the preceding claims, wherein step (c) is conducted at a pressure of about 10 to about 300 bar, and particularly at about 10 to about 140 bar.

7. The method of any of the preceding claims, wherein the pressure is kept substantially constant while conducting step (c).

8. The method of any of the preceding claims, wherein step (c) is conducted at a temperature of about 0 to about 60 °C.

9. The method of any of the preceding claims, wherein the hydrophilic excipient has a water solubility of at least about 2 wt.-% at 25 °C, and is preferably selected from the group of saccharides, proteins, and polymers.

10. The method of any of the preceding claims, wherein the first dense-phase extraction medium comprises a compressed gas or mixture of gases, in particular carbon dioxide, nitrogen, or air.

11. The method of any of the processing claims, wherein the first dense-phase extraction medium is a near critical or supercritical fluid.

12. The method of claim 4, wherein the second dense-phase extraction medium is substantially free of organic cosolvents.

13. The method of any of the preceding claims, wherein the colloidal particles are selected from liposomes, lipid complexes, solid lipid nanoparticles, lipoplexes, niosomes, micelles, and mixed micelles.

14. The method of any of the preceding claims, wherein the colloidal particles have an average diameter of about 20 nm to about 5 µm.

15. The method of any of the preceding claims, wherein the colloidal particles comprise an active ingredient.

16. The method of claim 15, wherein the active ingredient is a thermally labile compound, and preferably is a thermally labile peptide, polypeptide, protein, nucleic acid, cytotoxic agent, cytostatic agent, antimicrobial agent, antifungal agent, or antiviral agent.

17. Dry powder obtainable by the method according to any of the preceding claims.

18. A dry powder comprising solid particles of a hydrophilic excipient, wherein at least part of said particles individually comprise one or more incorporated colloidal particles and a thermally labile active compound.

19. A dry powder comprising solid particles of a hydrophilic excipient, wherein at least part of said particles individually comprise one or more incorporated thermally labile colloidal particles and optionally an active compound.

20. The dry powder of any of claims 17 to 19 having a residual water content of not more than about 7 wt.-%, and preferably not more than about 3 wt.-%.

21. Use of the dry powder of any of claims 17 to 20 for the manufacture of a medicament or a diagnostic product.

22. A pharmaceutical composition comprising the dry powder of any of claims 17 to 20.
